# EUROPEAN PATENT SPECIFICATION

(11) **EP 3 984 388 B1**
(45) Date of publication and mention of the grant of the patent: **04.12.2024**
(21) Application number: 21213373.0
(22) Date of filing: 26.10.2016
(51) Int. Cl.: A24F 40/465, A24D 1/20, A24F 40/20

(54) **APPARATUS FOR INDUCTIVE HEATING OF SMOKABLE MATERIAL**
VORRICHTUNG ZUR INDUKTIVEN ERWÄRMUNG VON RAUCHBAREM MATERIAL
APPAREIL DE CHAUFFAGE INDUCTIF DE MATIÈRE À FUMER

(30) Priority: 30.10.2015 US 201514927556
(43) Date of publication of application: 20.04.2022
(62) Divisional of application: 20205060.5
(73) Proprietor: Nicoventures Trading Limited, London WC2R 3LA (GB)
(72) Inventor: BLANDINO, Thomas, Cottage Grove, 53527 (US); WILKE, Andrew, Madison, 53704 (US); FRATER, James, Madison, 53718 (US)
(74) Representative: Dehns

(56) References cited:
- EP-A1- 2 316 286
- WO-A1-2015/117702
- WO-A1-95/27411
- US-A1- 2014 060 554

## Description

### Technical Field

The present invention relates to apparatus for heating smokable material to volatilise at least one component of the smokable material, to articles for use with such apparatus, to systems comprising such articles and apparatuses, to methods of manufacturing magnetic field generators for use in such apparatuses, and to methods of manufacturing heaters for use in heating smokable material.

### Background

Smoking articles such as cigarettes, cigars and the like burn tobacco during use to create tobacco smoke. Attempts have been made to provide alternatives to these articles by creating products that release compounds without combusting. Examples of such products are so-called "heat not burn" products or tobacco heating devices or products, which release compounds by heating, but not burning, material. The material may be, for example, tobacco or other non-tobacco products, which may or may not contain nicotine.

According to WO9527411A1, it is known to provide an induction heating source for use with an electrical smoking article. The induction heating source provides an alternating electromagnetic field which inductively heats a susceptor in thermal proximity with tobacco flavor medium to generate aerosols. A plurality of induction heaters are employed and/or the tobacco flavor medium is translated with respect to the induction heater or susceptor. The tobacco flavor medium can form an intimate structure with the susceptor and can take the form of a cylindrical cigarette or a web.

According to EP2316286A1, it is known to provide an electrically heated smoking system for receiving an aerosol-forming substrate. The system comprises at least one heater for heating the aerosol-forming substrate to form the aerosol, and a power supply for supplying power to the at least one heater. The at least one heater comprises one or more electrically conductive tracks on an electrically insulating substrate. In one arrangement, the one or more electrically conductive tracks have temperature coefficient of resistance characteristics such that the one or more electrically conductive tracks can act as resistive heaters and as a temperature sensor. In another arrangement, the electrically heated smoking system further comprises a thermally insulating material for insulating the at least one heater from the outside of the electrically heated smoking system.

According to WO2015117702A1, it is known to provide an aerosol-generating system comprising a fluid-permeable electric heater assembly, the heater assembly comprising: an electrically insulating substrate, an aperture being formed in the electrically insulating substrate; and a heater element having a first face fixed to the electrically insulating substrate, the heater element spanning the aperture and comprising a plurality of electrically conductive filaments connected to first and second electrically conductive contact portions, the first and second electrically conductive contact portions positioned on opposite sides of the aperture to one another, wherein the first and second electrically conductive contact portions are configured to allow contact with an external power supply.

According to US2014060554A1, it is known to provide an electronic smoking article comprising one or more microheaters and methods of forming an aerosol in a smoking article.

### Summary

According to the invention, there is provided an apparatus according to claim 1.

Optional features of the apparatus are set out in the dependent claims.

According to the invention, there is provided a system according to claim 13.

Optional features of the system are set out in the dependent claims.

Also disclosed is a method of manufacturing a heater for use in heating smokable material to volatilise at least one component of the smokable material, the method comprising:
providing a substrate; and forming a closed circuit of heating material on the substrate, wherein the heating material is heatable by penetration with a varying magnetic field, and wherein the forming comprises depositing the heating material on the substrate.

In an example, the forming comprises depositing the closed circuit of heating material on the substrate.

In an example, the depositing comprises printing.

In an example, the method comprises depositing a plurality of closed circuits of heating material on the substrate.

In an example, the method comprises printing the plurality of closed circuits of heating material on the substrate.

In an example, the depositing comprises depositing the plurality of closed circuits of heating material on the substrate so that the plurality of closed circuits are out of contact with each other.

In an example, the depositing comprises depositing the plurality of closed circuits of heating material on the substrate so that the plurality of closed circuits are arranged concentrically in relation to each other.

In an example, the heater is for use in an article disclosed herein.

Also disclosed is a method of manufacturing a magnetic field generator for use in apparatus for heating smokable material to volatilise at least one component of the smokable material, the method comprising:
providing a support; and
forming an electrically conductive coil on the support, wherein the forming comprises depositing electrically conductive material on the support.

In an example, the forming comprises depositing the electrically conductive coil on the support.

In an example, the depositing comprises printing.

In an example, the forming comprises forming the electrically conductive coil on the support so that the electrically conductive material bonds to the support.

In an example, the method comprises electrically connecting the electrically conductive coil to a device for passing a varying electrical current through the electrically conductive coil.

In an example, the method comprises:
forming a plurality of electrically conductive coils; and
connecting each of the plurality of electrically conductive coils to a device for passing a varying electrical current through the electrically conductive coils.

In an example, the connecting comprises connecting all of the plurality of electrically conductive coils to the same device.

Also disclosed is an article for use with apparatus for heating smokable material to volatilise at least one component of the smokable material, the article comprising:
smokable material; and
a film defining a closed circuit of heating material, wherein the heating material is heatable by penetration with a varying magnetic field to heat the smokable material.

In an example, the closed circuit of heating material is a printed closed circuit of heating material.

In an example, the closed circuit of heating material is a closed circuit of ink.

In an example, the article comprises one or more films comprising a plurality of closed circuits of heating material.

In an example, the plurality of closed circuits of heating material are arranged concentrically in relation to each other.

In an example, the heating material is in contact with the smokable material.

In an example, the article comprises a substrate, and the closed circuit of heating material is on the substrate.

In an example, the substrate comprises the smokable material.

In an example, the heating material comprises one or more materials selected from the group consisting of: an electrically-conductive material, a magnetic material, and a magnetic electrically-conductive material.

In an example, the heating material comprises a metal or a metal alloy.

In an example, the heating material comprises one or more materials selected from the group consisting of: aluminium, gold, iron, nickel, cobalt, conductive carbon, graphite, plain-carbon steel, stainless steel, ferritic stainless steel, copper, and bronze.

Also disclosed is apparatus for heating smokable material to volatilise at least one component of the smokable material, the apparatus comprising:
a magnetic field generator for generating a varying magnetic field for use in heating the smokable material,
wherein the magnetic field generator comprises a film defining a coil of electrically conductive material, and a device for passing a varying electrical current through the coil.

In an example, the coil is a printed coil.

In an example, the coil is a coil of ink.

In an example, the magnetic field generator comprises a support, and the coil is bonded to the support.

In an example, the coil is a two-dimensional spiral.

In an example, the magnetic field generator comprises one or more films defining a plurality of coils of electrically conducive material.

In an example, the plurality of coils are adjacent to each other on the support.

In an example, a first coil of the plurality of coils occupies a first area on a support, and a second coil of the plurality of coils occupies a second area on a support, wherein the second area is smaller than the first area.

In an example, each of the plurality of coils is connected to a respective device for passing a varying electrical current through the coil connected to that device.

In an example, the apparatus comprises a controller, wherein each of the respective devices is connected to the controller.

In an example, the controller is configured to control each of the respective devices independently to cause the generation of a plurality of respective varying magnetic fields.

In an example, the apparatus comprises an interface for cooperating with an article comprising the smokable material and heating material that is heatable by penetration with a varying magnetic field to heat the smokable material, and the magnetic field generator is configured so that the varying magnetic field penetrates the interface when the article is cooperating with the interface.

In an example, the apparatus comprises heating material that is heatable by penetration with a varying magnetic field to heat smokable material, wherein the magnetic field generator is configured so that the varying magnetic field penetrates the heating material of the apparatus.

Also disclosed is apparatus for heating smokable material to volatilise at least one component of the smokable material, the apparatus comprising:
a magnetic field generator for generating a varying magnetic field for use in heating the smokable material,
wherein the magnetic field generator comprises a coil in the form of a two-dimensional spiral of electrically conductive material, and a device for passing a varying electrical current through the coil.

The apparatus may have any one or more of the features of the above-described apparatuses.

Also disclosed is a system, comprising:
apparatus for heating smokable material to volatilise at least one component of the smokable material; and
an article for use with the apparatus, wherein the article comprises the smokable material;
wherein the apparatus comprises a magnetic field generator for generating a varying magnetic field for use in heating the smokable material, wherein the magnetic field generator comprises a film defining a coil of electrically conductive material, and a device for passing a varying electrical current through the coil.

In an example, the article comprises heating material that is heatable by penetration with the varying magnetic field to heat the smokable material, the apparatus comprises an interface for cooperating with the article, and the magnetic field generator is configured so that the varying magnetic field penetrates the heating material of the article when the article is cooperating with the interface.

In an example, the article of the system is the article herein disclosed. The article of the system may have any one or more of the features of the above-described article.

In an example, the apparatus comprises heating material that is heatable by penetration with the varying magnetic field to heat the smokable material, and the magnetic field generator is configured so that the varying magnetic field penetrates the heating material of the apparatus.

Also disclosed is a system, comprising:
apparatus for heating smokable material to volatilise at least one component of the smokable material; and
an article for use with the apparatus, wherein the article comprises smokable material and a film defining a closed circuit of heating material, wherein the heating material is heatable by penetration with a varying magnetic field to heat the smokable material;
wherein the apparatus comprises an interface for cooperating with the article, and a magnetic field generator for generating a varying magnetic field to be used in heating the heating material when the article is cooperating with the interface.

In an example, the apparatus of the system is the apparatus herein disclosed. The apparatus of the system may have any one or more of the features of the above-described apparatus.

### Brief Description of the Drawings

Examples and embodiments of the invention will now be described, by way of example only, with reference to the accompanying drawings, in which:
Figure 1 shows a schematic front view of a portion of an example of an article for use with apparatus for heating smokable material to volatilise at least one component of the smokable material;
Figure 2 shows a schematic cross-sectional view of the portion of the article of Figure 1;
Figure 3 shows a schematic cross-sectional view of a portion of an example of another article for use with apparatus for heating smokable material to volatilise at least one component of the smokable material;
Figure 4 shows a schematic cross-sectional view of an example of apparatus according to the invention for heating smokable material to volatilise at least one component of the smokable material;
Figure 5 shows a schematic cross-sectional view of an example of another apparatus for heating smokable material to volatilise at least one component of the smokable material;
Figure 6 shows a schematic front view of a portion of a magnetic field generator of the apparatus of Figure 4;
Figure 7 shows a schematic cross-sectional view of the portion of the magnetic field generator of Figure 6;
Figure 8 shows a schematic front view of a portion of an example of another magnetic field generator of apparatus for heating smokable material to volatilise at least one component of the smokable material;
Figure 9 is a flow diagram showing an example of a method of manufacturing a heater for use in heating smokable material to volatilise at least one component of the smokable material; and
Figure 10 is a flow diagram showing an example of a method of manufacturing a magnetic field generator for use in apparatus for heating smokable material to volatilise at least one component of the smokable material.

### Detailed Description

As used herein, the term "smokable material" includes materials that provide volatilised components upon heating, typically in the form of vapour or an aerosol. "Smokable material" may be a non-tobacco-containing material or a tobacco-containing material. "Smokable material" may, for example, include one or more of tobacco per se, tobacco derivatives, expanded tobacco, reconstituted tobacco, tobacco extract, homogenised tobacco or tobacco substitutes. The smokable material can be in the form of ground tobacco, cut rag tobacco, extruded tobacco, reconstituted tobacco, reconstituted smokable material, liquid, gel, gelled sheet, powder, or agglomerates, or the like. "Smokable material" also may include other, non-tobacco, products, which, depending on the product, may or may not contain nicotine. "Smokable material" may comprise one or more humectants, such as glycerol or propylene glycol.

As used herein, the term "heating material" or "heater material" refers to material that is heatable by penetration with a varying magnetic field.

As used herein, the terms "flavour" and "flavourant" refer to materials which, where local regulations permit, may be used to create a desired taste or aroma in a product for adult consumers. They may include extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, gel, powder, or the like.

Induction heating is a process in which an electrically-conductive object is heated by penetrating the obj ect with a varying magnetic field. The process is described by Faraday's law of induction and Ohm's law. An induction heater may comprise an electromagnet and a device for passing a varying electrical current, such as an alternating current, through the electromagnet. When the electromagnet and the object to be heated are suitably relatively positioned so that the resultant varying magnetic field produced by the electromagnet penetrates the object, one or more eddy currents are generated inside the object. The object has a resistance to the flow of electrical currents. Therefore, when such eddy currents are generated in the object, their flow against the electrical resistance of the object causes the obj ect to be heated. This process is called Joule, ohmic, or resistive heating. An object that is capable of being inductively heated is known as a susceptor.

It has been found that, when the susceptor is in the form of a closed circuit, magnetic coupling between the susceptor and the electromagnet in use is enhanced, which results in greater or improved Joule heating.

Magnetic hysteresis heating is a process in which an object made of magnetic material is heated by penetrating the object with a varying magnetic field. A magnetic material can be considered to comprise many atomic-scale magnets, or magnetic dipoles. When a magnetic field penetrates such material, the magnetic dipoles align with the magnetic field. Therefore, when a varying magnetic field, such as an alternating magnetic field, for example as produced by an electromagnet, penetrates the magnetic material, the orientation of the magnetic dipoles changes with the varying applied magnetic field. Such magnetic dipole reorientation causes heat to be generated in the magnetic material.

When an object is both electrically-conductive and magnetic, penetrating the object with a varying magnetic field can cause both Joule heating and magnetic hysteresis heating in the object. Moreover, the use of magnetic material can strengthen the magnetic field, which can intensify the Joule heating.

In each of the above processes, as heat is generated inside the object itself, rather than by an external heat source by heat conduction, a rapid temperature rise in the object and more uniform heat distribution can be achieved, particularly through selection of suitable object material and geometry, and suitable varying magnetic field magnitude and orientation relative to the object. Moreover, as induction heating and magnetic hysteresis heating do not require a physical connection to be provided between the source of the varying magnetic field and the object, design freedom and control over the heating profile may be greater, and cost may be lower.

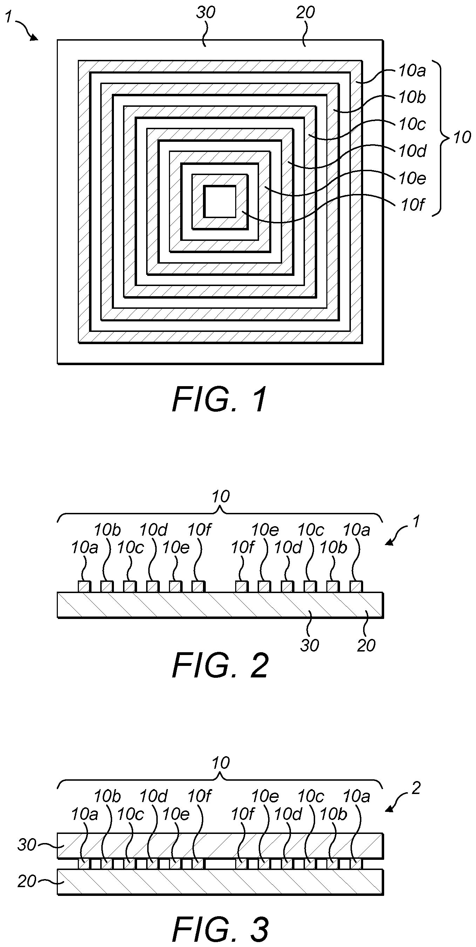

Referring to Figures 1 and 2 there are respectively shown a schematic front view and a schematic cross-sectional view of a portion of an article 1 according to an example. The article 1 is for use with apparatus for heating smokable material to volatilise at least one component of the smokable material without burning the smokable material. In this example, the article 1 comprises a substrate 20 and one or more films defining a plurality of closed circuits 10a-f of heating material 10. The heating material 10 is heatable by penetration with a varying magnetic field.

A thickness of the, or each, film defining the closed circuits 10a-f of heating material 10 may be no more than 1 micron, such as below 1 micron. In other examples, the thickness of the film may be more than 1 micron, such as more than 10 microns or more than 100 microns.

In this example, the substrate 20 comprises smokable material 30, such as tobacco. In some examples, the substrate 20 may comprise or consist entirely, or substantially entirely, of the smokable material 30, e.g. tobacco, such as reconstituted smokable material, e.g. reconstituted tobacco. The latter is sometimes referred to as "tobacco recon".

In this example, the plurality of closed circuits 10a-f of heating material 10 are heatable in use to heat the smokable material 30 to volatilise at least one component of the smokable material 30. Each of the closed circuits 10a-f of heating material 10 may be considered a heater for use in heating smokable material.

In some examples, the article 1 may comprise only one closed circuit 10a of heating material 10. In such examples, the closed circuit 10a of heating material 10 is heatable in use to heat smokable material 30 to volatilise at least one component of the smokable material 30. In other examples, such as that illustrated, the article 1 may comprise more than one closed circuit 10a-10f of heating material 10.

In this example, each of the plurality of closed circuits 10a-f of heating material 10 is square or rectangular. In other examples, each of the plurality of closed circuits 10a-f may be of any shape that defines a path that starts and ends at the same point so as to create a loop, such as circular or elliptical.

In this example, each of the plurality of closed circuits 10a-f of heating material 10 has a uniform width and a uniform thickness. In other examples, each of the plurality of closed circuits 10a-f of heating material may be different to at least one other of the closed circuits 10a-f. For example, the closed circuits 10a-f may have different widths of thicknesses. In other examples, a single closed circuit 10a may have a varying thickness or width along its path. Such variations in the width or thickness of the closed circuits 10a-f of heating material 10 can focus the heating of the heating material 10, which can result in a variation in the rate at which the heating material 10 volatilises the smokable material 30.

In some examples, the closed circuits 10a-f of heating material 10 can result in magnetic coupling between the closed circuits 10a-f of heating material 10 and an electromagnet of the apparatus in use being enhanced, which results in greater or improved Joule heating.

In this example, and indeed in all examples discussed herein, the heating material 10 is aluminium. However, in other examples, the heating material 10 may comprise one or more materials selected from the group consisting of: an electrically-conductive material, a magnetic material, and a non-magnetic material. In some examples, the heating material 10 may comprise a metal or a metal alloy. In some examples, the heating material 10 may comprise one or more materials selected from the group consisting of: aluminium, gold, iron, nickel, cobalt, conductive carbon, graphite, plain-carbon steel, stainless steel, ferritic stainless steel, copper, and bronze. Other heating material(s) may be used in other examples. It has also been found that, when magnetic electrically-conductive material is used as the heating material 10, magnetic coupling between the magnetic electrically-conductive material and an electromagnet of the apparatus in use may be enhanced. In addition to potentially enabling magnetic hysteresis heating, this can result in greater or improved Joule heating of the heating material 10, and thus greater or improved heating of the smokable material 30.

In this example, each of the plurality of closed circuits 10a-f of heating material 10a-f is in contact with the substrate 20. In some examples, the heating material 10 may be in the form of an ink. The closed circuits 10a-f of heating material 10 may thus be deposited directly on the substrate 20, for example by printing. Printing ink comprising heating material 10 on the substrate 20 may result in close integration of the heating material 10 with the substrate 10, which may result in good thermal transfer between the heating material 10 and the smokable material 30 comprised in the substrate 20.

Ink and films may have a small thickness. Therefore, induced electrical current and/or induced reorientation of magnetic dipoles in the ink or film when subjected to a varying magnetic field may penetrate most or all of the ink or film, rather than be confined to just a "skin" thereof as can be the case when a component comprising heating material has too great a thickness. Thus, a more efficient use of material is achieved and, in turn, costs are reduced.

In some examples, the depositing may result in the formation of the closed circuits 10a-f. In other examples, this may not be the case. For example, a film of heating material 10 may be deposited on the substrate 20, and then the closed circuits 10a-f of heating material 10 may be formed from the film, for example by etching the film.

In this example, the plurality of closed circuits 10a-f of heating material 10 are out of contact with each other. That is, they do not touch each other. In other examples, one or more of the plurality of closed circuits 10a-f may be in contact with one or more others of the plurality of closed circuits 10a-f.

In this example, the plurality of closed circuits 10a-f of heating material 10 are arranged concentrically in relation to each other. In other examples, the plurality of closed circuits 10a-f of heating material 10 may be arranged so that each of the closed circuits 10a-f is outside of each other of the closed circuits 10a-f, or in any other arrangement.

In this example, heating material 10 is deposited on an initially flat substrate 20. In this example, the substrate 20 and the plurality of closed circuits 10a-f of heating material 10 are together flexible or malleable. By "malleable" it is meant that article 1 is able to be pressed, bent, rolled, folded or flexed so as to take on different overall shapes without breaking for cracking, for example a cylindrical shape. The degree of flexibility depends on the material and thickness of the substrate 20, and the composition of the closed circuits 10a-f of heating material 10. Such flexibility may increase the versatility of the article 1, for example by increasing the number of plausible configurations for the article 1. Such constructions may be suitable for use in articles of a variety of different shapes. For example, the substrate 20 may be a layer on a surface of an article, may define a recess in an article, or may be flexed to fit into a recess in an article. In other examples, the substrate 20 and the plurality of closed circuits 10a-f of heating material 10 together may be substantially rigid.

In this example, the substrate 20 is substantially planar. In some examples, the substrate 20 may instead be non-planar, such as tubular. The closed circuits 10a-f of heating material 10 would then be on a surface of the tubular substrate 20. In other examples, the substrate 20 may be any other shape, for example conical.

In this example, the plurality of closed circuits 10a-f of heating material 10 are bonded to the substrate 20. The bonding may be achieved, for example, by a process of printing the heating material 10, or by adhering the heating material 10 to the substrate 20 using an adhesive. In other examples, the bonding may be achieved by a deposition process involving physical locking or intermingling of the heating material 10 and the substrate 20, or the heating material 10 and the smokable material 30. In some examples, when the deposition process comprises printing, a bond may be achieved by partial absorption of ink by the substrate 20. In examples in which the substrate 20 comprises the smokable material 30, such bonding of the heating material 10 to the substrate 20 may result in better thermal conduction from the heating material 10 to the substrate 20, and thus a higher proportion of the smokable material 30 being volatilised in use.

Referring to Figure 3 there is shown a schematic cross-sectional view of an example of another article according to an example. The article 2 is identical to the article 1 of Figures 1 and 2, except that the substrate 20 of the article 2 of Figure 3 does not comprise smokable material 30. Instead, the smokable material 30 is separate to substrate 20.

In this example, the substrate comprises paper or card. However, in some examples, the substrate 20 may additionally or alternatively comprise thermal insulation. Such thermal insulation can help to increase the proportion of heat which heats the smokable material 30 when the heating material 10 is heated by penetration with a varying magnetic field.

In this example, the smokable material 30 is comprised in a layer on the plurality of closed circuits 10a-f of heating material 10 and may, for example, be a layer of tobacco recon. That is, the closed circuits 10a-f of heating material 10 are arranged between the substrate 20 and the smokable material 30. In other examples, the smokable material 30 may be positioned on the substrate 20, and surrounding, at least in part, each of the plurality of closed circuits 10a-f of heating material 10. Each of the closed circuits 10a-f of heating material 10, and indeed the combination of the substrate 20 and the plurality of closed circuits 10a-f of heating material 10, may be considered a heater for use in heating smokable material.

In some examples, which may be respective variations to the examples discussed above, the article 1, 2 may comprise a mouthpiece defining a passageway that is in fluid communication with the smokable material 30. The mouthpiece may be made of any suitable material, such as a plastics material, cardboard, cellulose acetate, paper, metal, glass, ceramic, or rubber. In use, when the smokable material 30 is heated, volatilised components of the smokable material 30 can be readily inhaled by a user. In examples in which the article is a consumable article, once all or substantially all of the volatilisable component(s) of the smokable material 30 in the article has/have been spent, the user may dispose of the mouthpiece together with the rest of the article. This can be more hygienic than using the same mouthpiece with multiple articles, can help ensure that the mouthpiece is correctly aligned with the smokable material, and presents a user with a clean, fresh mouthpiece each time they wish to use another article. The mouthpiece, when provided, may comprise or be impregnated with a flavourant. The flavourant may be arranged so as to be picked up by heated vapour as the vapour passes through the passageway of the mouthpiece in use.

Each of the above-described articles 1, 2 and described variants thereof is usable with apparatus for heating the smokable material 30 to volatilise at least one component of the smokable material 30. When preparing the article 1, 2 for use with the apparatus, the article 1, 2 may first be rolled by a user so as to take on a substantially cylindrical shape. In some examples, the article 1, 2 may be provided to a user in a pre-rolled state. The apparatus may be to heat the smokable material 30 to volatilise the at least one component of the smokable material 30 without burning the smokable material 30. Example such apparatuses are described below.

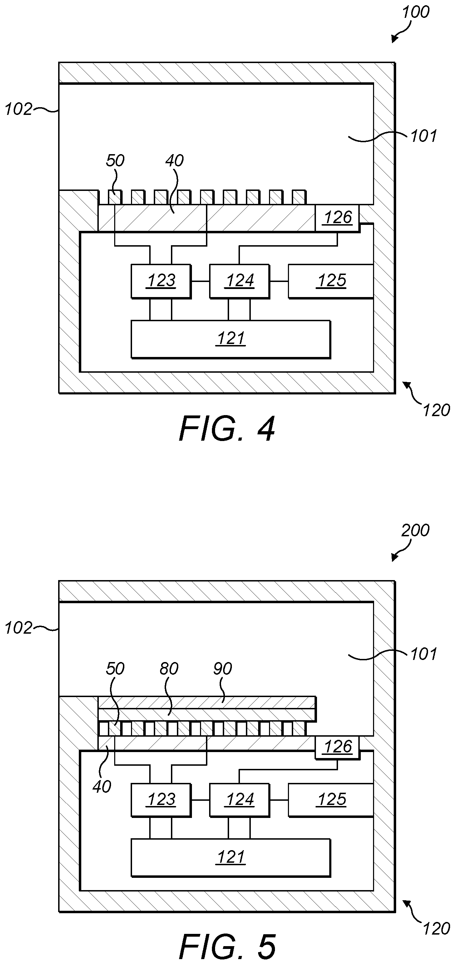

Referring to Figure 4 there is shown an example according to the invention of apparatus for heating smokable material to volatilise at least one component of the smokable material. The apparatus 100 is for use with an article comprising smokable material 30 and heating material 10, such as one of the articles 1, 2 discussed above.

The apparatus 100 of this embodiment comprises a magnetic field generator 120. The magnetic field generator 120 comprises an electrical power source 121, a film defining a coil 50 on a support 40, a device 123 for passing a varying electrical current, such as an alternating current, through the coil 50, a controller 124, a user interface 125 for user-operation of the controller 124, a temperature sensor 126, and an interface 101 for cooperating with the article.

A thickness of the film defining the coil 50 of electrically conductive material is no more than 1 micron, such as below 1 micron. In other examples, the thickness of the film may be more than 1 micron, such as more than 10 microns or more than 100 microns.

In this embodiment, the interface 101 comprises a recess 101 that is configured to receive the article via the opening 102. The recess 101 is configured to release the article via an opening 102 of the apparatus 100 after use of apparatus 100. The article may be released from the recess 101 by a user and replaced by another article for repeated use of the apparatus 100.

In this embodiment, the electrical power source 121 is a rechargeable battery. In other embodiments, the electrical power source 121 may be other than a rechargeable battery, such as a non-rechargeable battery, a capacitor, a battery-capacitor hybrid, or a connection to a mains electricity supply.

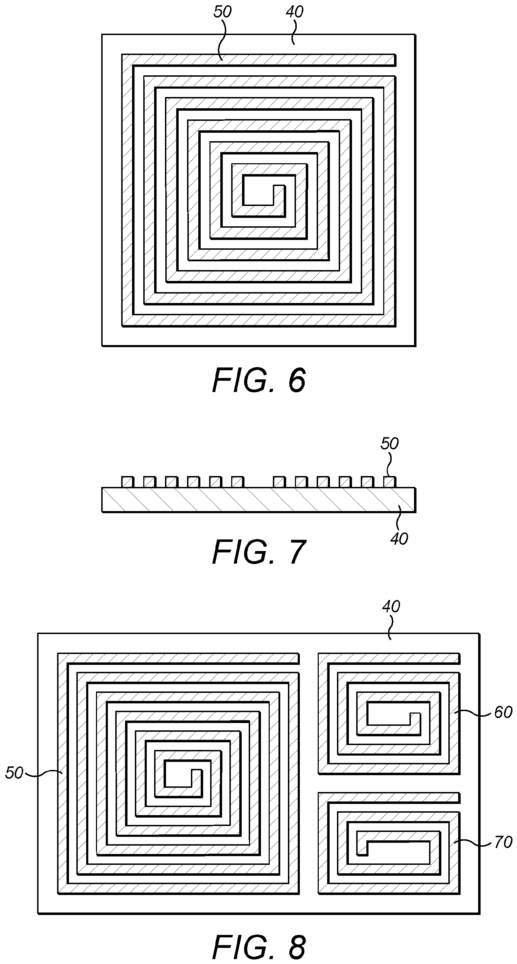

Referring to Figures 6 and 7, there are respectively shown a schematic front view and a schematic cross-sectional view of the coil 50 and support 40 of the apparatus 100 of Figure 4.

In this embodiment, the coil 50 is a two-dimensional spiral on a surface of the support 40. The coil 50 is defined by a film. In this embodiment, the support 40 is a non-electrically conductive support 40. That is, the support 40 is an electrical insulator. In other examples, the support 40 may be omitted.

In some embodiments, the coil 50 is deposited on a flat support 40. In this embodiment, the support 40 and the film defining coil 50 are together flexible or malleable. By "malleable" it is meant that an assembly of the support 40 and the film defining coil 50 is able to be pressed, bent, rolled, folded or flexed so as to take on different overall shapes without breaking for cracking, for example a cylindrical shape. The degree of flexibility depends on the material and thickness of the support 40, and the composition of the electrically conductive material of the coil 50. By altering the shape of an assembly of the support 40 and the film defining the coil 50 so that it has a three-dimensional shape, a three-dimensional transverse flux design may occur, when a varying electrical current is passed through the coil 50. Such a three-dimensional transverse flux design increases the number of plausible configurations for apparatus 100. For example, the support 40 may be a layer on a surface of the apparatus 100, may define a recess in the apparatus 100, or may be flexed to fit into a recess in the apparatus 100.

In this embodiment, the coil 50 is an electrically conductive coil configured to conduct a varying electrical current. In this embodiment, the electrically conductive material of the coil 50 is an electrically conductive film in the form of ink. The coil 50 of this embodiment thus comprises electrically conductive material.

In this embodiment, the coil 50 is in contact with the support 40. The coil 50 may be deposited directly on support 40. Depositing directly on the support 40 may result in a close integration of the electrically conductive ink with the support 40, which may better bind the coil 50 to the substrate 40 and help to avoid delamination. The depositing may, for example, comprise printing.

In some embodiments, the depositing may result in the formation of the coil 50. In other embodiments, this may not be the case. For example, a film of electrically conductive material may be deposited on the support 40, and the coil 50 may be formed from the film, for example by etching the film.

In this embodiment, the coil 50 is bonded to the support 40. The bonding may be achieved by, for example, printing or chemically or mechanically adhering the coil 50 to the support 40. In other embodiments, the bonding may be achieved by a deposition process involving physical locking or intermingling of the coil 50 and the support 40. In some embodiments, when the deposition process comprises printing, a bond may be achieved by partial absorption of ink by the support 40.

In this embodiment, the coil 50 is a two-dimensional spiral. In this embodiment, coil 50 is a generally square or rectangular coil. In other embodiments, the coil 50 may have a different shape, such as generally circular or elliptical. In some embodiments, the coil 50 may be a three-dimensional spiral. In some such embodiments, the coil 50 may be manufactured using an additive manufacturing technique, such as 3D printing.

In this embodiment, adjacent spaced portions of the coil 50 are regularly spaced. In other embodiments, such portions of the coil 50 may not be regularly spaced. Relatively-closely spaced portions of the coil 50 may create a denser magnetic flux in use than less-closely-spaced portions of the coil 50. Such a structure may enable progressive heating of smokable material, and thereby progressive generation of vapour, to be achieved.

In this embodiment, the combination of the support 40 and the coil 50 is flexible. The degree of flexibility depends on the material and thickness of each of the support 40 and the coil 50. In other embodiments, the combination of the support 40 and the coil 50 may be relatively rigid. By providing that the combination of the support 40 and the coil 50 is flexible, the combination of the support 40 and the coil 50 may be fitted into an irregularly-shaped space in the apparatus 100. Further, by providing that the combination of the support 40 and the coil 50 is flexible, the combination of the support 40 and the coil 50 may be more resistant to damage.

With reference once again to Figure 4, it will be seen that in this embodiment the combination of the support 40 and the coil 50 define part of the recess 101. In other embodiments, a protective structure may be provided between the combination of the support 40 and the coil 50 and the recess 101, to help protect the support 40 and the coil 50 from damage during use of the apparatus 100.
In this embodiment, the device 123 for passing a varying electrical current through the coil 50 is electrically connected between the electrical power source 121 and the coil 50. In this embodiment, the controller 124 also is electrically connected to the electrical power source 121, and is communicatively connected to the device 123. More specifically, in this embodiment, the controller 124 is for controlling the device 123, so as to control the supply of electrical power from the electrical power source 121 to the coil 50. In this embodiment, the controller 124 comprises an integrated circuit (IC), such as an IC on a printed circuit board (PCB). In other embodiments, the controller 124 may take a different form. In some embodiments, the apparatus may have a single electrical or electronic component comprising the device 123 and the controller 124. The controller 124 is operated in this embodiment by user-operation of the user interface 125. In this embodiment, the user interface 125 is located at the exterior of the apparatus 100. The user interface 125 may comprise a push-button, a toggle switch, a dial, a touchscreen, or the like. In other embodiments, the user interface 125 may be remote and connected to the rest of the apparatus wirelessly, such as via Bluetooth.

In this embodiment, operation of user interface 125 by a user causes the controller 124 to cause the device 123 to cause a varying electrical current to pass through the coil 50, so as to cause the coil 50 to generate a varying magnetic field. When the article 1, 2 is located in the recess 101, the coil 50 of the apparatus 100 and the heating material 10 of the article 1, 2 are suitably relatively positioned so that the alternating magnetic field produced by the coil 50 penetrates the heating material 10 of the article 1, 2. When the heating material 10 of the article 1, 2 is an electrically-conductive material, this may cause the generation of one or more eddy currents in the heating material 10. The flow of eddy currents in the heating material 10 against the electrical resistance of the heating material 10 causes the heating material 10 to be heated by Joule heating. As mentioned above, when the heating material 10 is made of a magnetic material, the orientation of magnetic dipoles in the heating material 10 changes with the changing applied magnetic field, which causes heat to be generated in the heating material 10.

The apparatus 100 of this embodiment comprises a temperature sensor 126 for sensing a temperature of the recess 101. The temperature sensor 126 is communicatively connected to controller 124, so that controller 124 is able to monitor the temperature of the recess 101. In some embodiments, the temperature sensor 126 may be arranged to take an optical temperature measurement of the recess 101 or article 1, 2. In some embodiments, the article to be located in the recess 101 may comprise a temperature detector, such as a resistance temperature detector (RTD), for detecting a temperature of the article. The article may further comprise one or more terminals connected, such as electrically-connected, to the temperature detector. The terminal(s) may be for making connection, such as electrical connection, with a temperature monitor (not shown) of the apparatus 100 when the article is in recess 101. The controller 124 may comprise the temperature monitor. The temperature monitor of apparatus 100 may thus be able to determine a temperature of the article during use of the article with the apparatus 100.

In some embodiments, by providing that a component of the article 1, 2 comprising the heating material 10 has a suitable resistance, the response of the heating material 10 to a change in temperature could be sufficient to give information regarding temperature inside the article 1, 2. The temperature sensor 126 of the apparatus 100 may then comprise a probe for analysing the heating material.

On the basis of one or more signals received from temperature sensor 126 or temperature detector, the controller 124 may cause the device 123 to adjust a characteristic of the varying electrical current passed through the coil 50 as necessary, in order to ensure that the temperature of the recess 101, article 1,2 or heating material 10 remains within a predetermined temperature range. The characteristic may be, for example, amplitude or frequency. Within the predetermined temperature range, in use the smokable material 30 within an article 1, 2 located in the recess 101 is heated sufficiently to volatilise at least one component of the smokable material 30 without combusting the smokable material 30. Accordingly, the controller 124, and the apparatus 100 as a whole, is arranged to heat the smokable material 30 to volatilise the at least one component of the smokable material 30 without combusting the smokable material 30. In some embodiments, the temperature range is about 50°C to about 250°C, such as between about 50°C and about 150°C, between about 50°C and about 120°C, between 25 about 50°C and about 100°C, between about 50°C and about 80°C, or between about 60°C and about 70°C. In some embodiments, the temperature range is between about 170°C and about 220°C. In other embodiments, the temperature range may be other than these ranges. In some embodiments, the temperature sensor 126 may be omitted.

In some embodiments, the apparatus 100 may comprises a mouthpiece (not shown). The mouthpiece may be releasably engageable with the rest of apparatus 100 so as to connect the mouthpiece to the rest of apparatus 100. In other embodiments, the mouthpiece and the rest of apparatus 100 may be permanently connected, such as through a hinge or flexible member. The mouthpiece may be locatable so as to cover the opening 102 into the recess 101. When the mouthpiece is so located, a channel through the mouthpiece may be in fluid communication with the smokable material 30. In use, the channel acts as a passageway for permitting volatilised material to pass from the smokable material 30 to an exterior of apparatus 100. The mouthpiece, when provided, may comprise or be impregnated with a flavourant. The flavourant may be arranged so as to be picked up by heated vapour as the vapour passes through the passageway of the mouthpiece in use.

As the smokable material 30 in the article 1, 2 is being heated, a user may be able to inhale the volatilised component(s) of the smokable material 30 by drawing the volatilised component(s) through a mouthpiece of the article (when provided) or through a mouthpiece of the apparatus 100 (when provided). Air may enter the article via a gap between the article and apparatus 100, or in some embodiments apparatus 100 may define an air inlet that fluidly connects the smokable material 30 with the exterior of apparatus 100. As the volatilised component(s) are removed from the article, air may be drawn into smokable material 30 via the air inlet of apparatus 100.

Some embodiments of the apparatus 100 may be arranged to provide haptic feedback to a user. The feedback could indicate that heating is taking place, or be triggered by a timer to indicate that greater than a predetermined proportion of the original quantity of volatilisable component(s) of the smokable material 30 in an article in has/have been spent, or the like. The haptic feedback could be created by interaction of the coil 50 and the heating material 10, by interaction of an electrically-conductive element with the coil 50, by rotating an unbalanced motor, by repeatedly applying and removing a current across a piezoelectric element, or the like.

In some embodiments, the apparatus may comprise more than one coil. The plurality of coils of the apparatus could be operable to provide progressive heating of the smokable material in an article 1, 2, and thereby progressive generation of vapour. For example, one coil may be able to heat a first region of the heating material 10 relatively quickly to initialise volatilisation of at least one component of the smokable material and formation of a vapour in a first region of the smokable material. Another coil may be able to heat a second region of the heating material 10 relatively slowly to initialise volatilisation of at least one component of the smokable material and formation of a vapour in a second region of the smokable material. Accordingly, a vapour is able to be formed relatively rapidly for inhalation by a user, and vapour can continue to be formed thereafter for subsequent inhalation by the user even after the first region of the smokable material may have ceased generating vapour. The initially-unheated second region of smokable material could act as a heat sink, to reduce the temperature of created vapour or make the created vapour mild, during heating of the first region of smokable material.

Referring to Figure 8 there is shown a schematic front view of a structure comprising a support 40 and one or more films defining a plurality of coils 50, 60, 70 adjacent to each other on the support 40. The structure of Figure 8 may be usable in the apparatus 100 of Figure 4 in place of the structure of Figures 6 and 7.

In this example, each of the plurality of coils 50, 60, 70 comprises electrically conductive material. Each of the plurality of coils 50, 60, 70 may be provided on the support 40 using any of the processes described herein for the provision of the coil 50 on the support 40 of Figure 6.

While in this example the structure comprises first to third coils 50, 60, 70, in other examples the structure may comprise two coils or more than three coils of electrically conductive material.

In this example, each of the coils 50, 60, 70 occupies a respective area on the support 40. In this example, the first coil 50 occupies a first area, the second coil 60 occupies a second area which is smaller than the first area, and the third coil 70 occupies a third area. In this example, the second and third area are substantially equal. In other examples, the second and third areas may be of respective different sizes. In some examples, the coils 50, 60, 70 may occupy respective same-sized areas.

In use, each of the coils 50, 60, 70 may be used in heating respective different regions of heating material 10 of an article located in the recess 101. That is, the respective varying magnetic fields created by the coils 50, 60, 70 may penetrate different respective regions of the heating material 10. The different regions of the heating material 10 may be configured to heat respective different areas of smokable material 30 in the article that may, for example, comprise different flavourants and thereby release vapour of different respective flavours.

In some examples, each of the coils 50, 60, 70 may be connected to the same, common device 123 for passing respective varying electrical current through the coils 50, 60, 70. In other examples, the coils 50, 60, 70 may be connected to respective separate devices 123 for passing a varying electrical current through the coil 50, 60, 70 connected to the device 123.

In various examples, the device 123, or each of the devices 123, is connected to the controller 124. The controller 124 is configured to control the one, or each, device 123 to cause the generation of a plurality of respective varying magnetic fields. The controller 124 may be configured to control the device(s) 123 so as to control independently the varying magnetic fields output from the coils 50, 60, 70.

In some examples, the varying electrical current may be passed through the coils 50, 60, 70 simultaneously. This may allow a greater area of heating material 10 to be heated sufficiently at any one time, or may allow a smaller area of heating material 10 to be heated in a shortened period of time. In other examples, the varying electrical current may be passed through the coils 50, 60, 70 in a predetermined sequence. The coils 50, 60, 70 may be operable to provide progressive heating of the heating material 10, and thus progressive heating of the smokable material 30 in the article located in recess 101, so as to provide progressive generation of vapour, as described above.

In some examples, the controller 124 may be configured to control the device(s) 123 in such a way that the coils 50, 60, 70 are caused to output respective varying magnetic fields in a cyclical or peristaltic manner. In some examples, the cyclically or peristaltically output varying magnetic fields may heat respective portions of heating material 10 cyclically or peristaltically, so as to heat the vapour output from the smokable material 30 in a cyclical or peristaltic manner. This may cause movement of the vapour in a predetermined direction, such as towards an outlet of the apparatus 100 and thus towards a user at the outlet.

In the embodiment of Figure 4, the apparatus 100 is for use with an article 1, 2 that itself comprises heating material 10 that is heatable by penetration with a varying magnetic field. However, in some embodiments, the apparatus may additionally or alternatively comprise such heating material.

Referring to Figure 5, there is shown a schematic cross-sectional view of an example of apparatus for heating smokable material to volatilise at least one component of the smokable material. The apparatus 200 of this example is for use with an article comprising smokable material 30. The apparatus 200 is substantially similar to apparatus 100, except that it further comprises thermal insulation 80 and heating material 90.

In this example, the thermal insulation 80 is located between the coil 50 and the heating material 90. The thermal insulation 80 may comprise, for example, one or more materials selected from the group consisting of: aerogel, vacuum insulation, wadding, fleece, non-woven material, non-woven fleece, woven material, knitted material, nylon, foam, polystyrene, polyester, polyester filament, polypropylene, a blend of polyester and polypropylene, cellulose acetate, paper or card, and corrugated material such as corrugated paper or card. The thermal insulation 80 may additionally or alternatively comprise an air gap. Such thermal insulation 80 may help to prevent heat loss from the heating material 90 to components of the apparatus 200, may help to increase heating efficiency of the smokable material 30 of the article 1, 2 in the recess 101, and/or may help to reduce the transfer of heating energy from the heating material 90 to an outer surface of apparatus 200. This may improve the comfortableness with which a user is able to hold apparatus 200.

In some examples, the coil 50 may be embedded in the thermal insulation 80. The thermal insulation 80 may abut or envelop the coil 50. In addition to the thermal benefits discussed above, such a configuration may help to increase the robustness of the apparatus 200, such as by helping to maintain the relative positioning of the coil 50 and the recess 101.

In some examples, the thermal insulation 80 may be omitted.

In this example, the recess 101 is partially defined by the heating material 90.

In some examples, the heating material 90 may comprise deposited heating material 10 or ink. The heating material 90 may be deposited on the thermal insulation 80, for example, by printing. The heating material 90 may comprise at least one closed circuit of heating material, which may provide the benefits described elsewhere herein.

The inclusion of the heating material 90 in the apparatus 200 reduces the required complexity of an article for use with the apparatus 200. Heating material may be used repeatedly for heating smokable material 30, and thus it may be an efficient use of heating material to include the heating material 90 in the apparatus 200 rather than in a consumable article for use with the apparatus 200.

An impedance of the coil 50 of this example is equal, or substantially equal, to an impedance of the heating material 90. If the impedance of the heating material 90 were instead lower than the impedance of the coil 50, then the voltage generated across the heating material 90 in use may be lower than the voltage that may be generated across the heating material 90 when the impedances are matched. Alternatively, if the impedance of the heating material 90 were instead higher than the impedance of the coil 50, then the electrical current generated in the heating material 90 in use may be lower than the current that may be generated in heating material 90 when the impedances are matched. Matching the impedances may help to balance the voltage and current to maximise the heating power generated at the heating material 90 when heated in use. However, in some other examples, the impedances may not be matched.

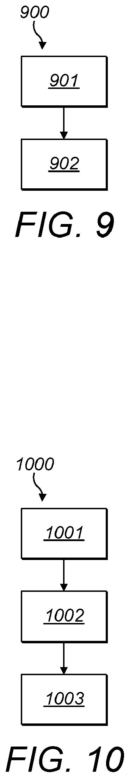

Referring to Figure 9 there is shown a flow diagram of an example method of manufacturing a heater for use in heating smokable material to volatilise at least one component of the smokable material.

Broadly speaking, the method 900 of this example comprises providing 901 a substrate, and forming 902 a closed circuit of heating material on the substrate. The forming comprises depositing the heating material. The heating material 10 is heatable by penetration with a varying magnetic field.

A closed circuit of heating material may be of any shape that defines a path that starts and ends at the same point so as to create a loop.

In this example, the substrate comprises smokable material. In other examples, the substrate may be free of smokable material. In some examples, the method 900 may comprise a step of providing smokable material, such as on the substrate or on the heating material.

In this example, the forming 902 comprises depositing the closed circuit of heating material on the substrate. However, in other examples, the forming 902 may comprise depositing a film of heating material, and then forming the closed circuit 10a of heating material from the film, for example by etching the film.

The heating material may be in the form of an ink. The heating material may be suitable for use in an additive manufacturing technique, such as 3D printing. The use of an ink may help to ensure that the closed circuit is of a pre-determined structure and of an even thickness on the substrate. The use of an ink also can result in an efficient use of heating material. Other benefits of using ink are discussed elsewhere herein.

In some examples, the forming 902 comprises forming a plurality of closed circuits of heating material. In such examples, each of the plurality of closed circuits of heating material may be of any shape that defines a path that starts and ends at the same point so as to create a loop.

The plurality of closed circuits of heating material may be arranged so that they are out of contact with each other. That is, they do not touch each other. In other examples, one or more of the plurality of closed circuits may be in contact with one or more others of the plurality of closed circuits. In some examples, the plurality of closed circuits of heating material are arranged concentrically in relation to each other. In other examples, the plurality of closed circuits of heating material may be formed so that each of the closed circuits is outside of each other of the closed circuits, or in any other arrangement.

Referring to Figure 10 there is shown a flow diagram showing an example of a method of manufacturing a magnetic field generator for use in apparatus for heating smokable material to volatilise at least one component of the smokable material.

Broadly speaking, the method 1000 of this example comprises providing 1001 a support, forming 1002 an electrically conductive coil on the support, wherein the forming comprises depositing electrically conductive material on the support, so that the electrically conductive material bonds to the support, and electrically connecting 1003 the coil to a device for passing a varying electrical current through the coil. However, in other examples, step 1001 and/or step 1003 may be omitted.

Preferably, the support is non-electrically conductive. That is, support is an electrical insulator. However, in some examples, bonding of the electrically conductive material to the support may be omitted.

As noted above, the forming comprises depositing electrically conductive material. In some examples, the depositing results in the formation of the coil. In other examples, this may not be the case. For example, a film of electrically conductive material may be deposited, and then the coil may be formed from the film, for example by etching the film. In some examples, the forming 1002 may comprise forming a plurality of electrically conductive coils, wherein the forming comprises depositing electrically conductive material. In some examples the forming 1002 may comprise forming two coils, but in other examples the number of coils formed may be three of more. In some examples, the plurality of coils may have identical geometries. In other examples, the coils may have different geometries. In some examples, some or all of the coils occupy differently-sized areas on the substrate.

In some examples, the connecting 1003 may comprise connecting each of the plurality of coils to a device for passing a varying electrical current through the electrically conductive coils. In some examples, the connecting 1003 may comprise connecting each of coils to a respective device for passing a varying electrical current through the electrically conductive coil connected to that device.

In each of the above-discussed examples, the film comprising heating material 10 is deposited in a method comprising printing. However, in other examples, the film could be deposited by a different method, such as sputtering, evaporation, chemical vapour deposition, molecular beam epitaxy, electroplating, screen printing, laser etching, drying, firing, curing, and the like.

In each of the above-discussed examples, the film defining the coil 50 of electrically conductive material is deposited in a method comprising printing. However, in other examples, the film could be deposited by a different method, such as sputtering, evaporation, chemical vapour deposition, molecular beam epitaxy, electroplating, screen printing, laser etching, drying, firing, curing, and the like.

In each of the examples discussed above, the heating material 10 may have a skin depth, which is an exterior zone within which most of an induced electrical current and/or induced reorientation of magnetic dipoles occurs. By providing that the component comprising the heating material 10 has a relatively small thickness, a greater proportion of the heating material 10 may be heatable by a given varying magnetic field, as compared to heating material in a component having a depth or thickness that is relatively large as compared to the other dimensions of the component. Thus, a more efficient use of material is achieved. In turn, costs are reduced.

In some examples, a component comprising the heating material 10 may comprise discontinuities or holes therein. Such discontinuities or holes may act as thermal breaks to control the degree to which different regions of the smokable material are heated in use. Areas of the heating material 10 with discontinuities or holes therein may be heated to a lesser extent that areas without discontinuities or holes. This may help progressive heating of the smokable material, and thus progressive generation of vapour, to be achieved. Such discontinuities or holes may, on the other hand, be used to optimise the creation of complex eddy currents in use.

In each of the above described embodiments and examples, the smokable material comprises tobacco. However, in respective variations to each of these embodiments and examples, the smokable material may consist of tobacco, may consist substantially entirely of tobacco, may comprise tobacco and smokable material other than tobacco, may comprise smokable material other than tobacco, or may be free of tobacco. In some embodiments and examples, the smokable material may comprise a vapour or aerosol forming agent or a humectant, such as glycerol, propylene glycol, triacetin, or diethylene glycol.

An article embodying the present invention may be a cartridge, for example.

In each of the above described embodiments and examples, the article 1, 2 is a consumable article. Once all, or substantially all, of the volatilisable component(s) of the smokable material of the article 1, 2 has/have been spent, the user may dispose of the article 1, 2. The user may subsequently re-use the apparatus with another of the articles 1, 2. However, in other respective embodiments and examples, the article 1, 2 may be non-consumable, and the apparatus and the article 1, 2 may be disposed of together once the volatilisable component(s) of the smokable material has/have been spent.

In some embodiments and examples, an article 1, 2 as discussed above is sold, supplied or otherwise provided separately from apparatus 100, with which it is usable. However in some embodiments and examples, the apparatus 100 and one or more of the articles 1, 2 may be provided together as a system, such as a kit or an assembly, possibly with additional components, such as cleaning utensils.

The invention could be implemented in a system comprising any one of the articles discussed herein, and any one of the apparatuses discussed herein, wherein the apparatus itself has heating material, such as in a susceptor, for heating by penetration with the varying magnetic field generated by the magnetic field generator. Heat generated in the heating material of the apparatus could be transferred to the article to heat, or further heat, the smokable material therein when the portion of the article is in the recess 101.

## Claims

1. Apparatus for heating smokable material to volatilize at least one component of the smokable material, the apparatus comprising:
a magnetic field generator (120) for generating a varying magnetic field for use in heating the smokable material,
wherein the magnetic field generator (120) comprises a film defining a coil (50) of electrically conductive material, and a device (123) for passing a varying electrical current through the coil (50),
wherein the magnetic field generator (120) comprises a support (40) and wherein the coil (50) is bonded to the support (40).

2. An apparatus as claimed in claim 1, wherein a thickness of the coil (50) is no more than 1 µm.

3. An apparatus as claimed in claim 1 or 2, wherein the magnetic field generator (120) further comprises a controller (124) and a user interface (125) for user-operation of the controller (124).

4. An apparatus as claimed in claim 3, wherein the user interface (125) comprises a push button located on an exterior of the apparatus (100).

5. An apparatus as claimed in any preceding claim, wherein the magnetic field generator (120) further comprises a temperature sensor (126).

6. An apparatus as claimed in any preceding claim, wherein the magnetic field generator (120) further comprises an interface (101) for cooperating with an article (1,2).

7. An apparatus as claimed in claim 6, wherein the interface (101) comprises a recess (101) configured to receive the article (1,2) via an opening (102).

8. An apparatus as claimed in any preceding claim, wherein the magnetic field generator (120) comprises an electrical power source (121).

9. An apparatus as claimed in claim 8, wherein the electrical power source (121) comprises a rechargeable battery.

10. An apparatus as claimed in any preceding claim, wherein the support (40) is an electrical insulator.

11. An apparatus as claimed in any preceding claim, wherein an assembly of the support (40) and the film defining the coil (50) are flexible so as to be able to be pressed to form a cylindrical shape.

12. An apparatus as claimed in any preceding claim, wherein the coil (50) is bonded to the support (40) by chemically or mechanically adhering the coil (50) to the support (40).

13. A system comprising:
apparatus as claimed in any preceding claim; and
an article (1,2) for use with the apparatus, wherein the article (1,2) comprises the smokable material (30).

14. A system as claimed in claim 13, wherein the smokable material (30) comprises a tobacco containing material.

15. A system as claimed in claim 13 or 14, wherein the article (1,2) comprises heating material (10) that is heatable by penetration with the varying magnetic field to heat the smokable material (30).

16. A system as claimed in claim 15, wherein the heating material (10) comprises a metal or a metal alloy.

17. A system as claimed in claim 16, wherein the heating material (10) comprises one or more materials selected from the group consisting of: aluminium, gold, iron, nickel, cobalt, conductive carbon, graphite, plain-carbon steel, stainless steel, ferritic stainless steel, copper and bronze.

## Patentansprüche

1. Einrichtung zum Erhitzen von rauchbarem Material, um mindestens einen Bestandteil des rauchbaren Materials zu verflüchtigen, wobei die Einrichtung umfasst:
einen Magnetfeldgenerator (120) zum Erzeugen eines sich verändernden Magnetfelds zur Verwendung beim Erhitzen des rauchbaren Materials,
wobei der Magnetfeldgenerator (120) einen Film, der eine Spule (50) aus elektrisch leitfähigem Material definiert, und eine Vorrichtung (123) zum Leiten eines sich verändernden elektrischen Stroms durch die Spule (50) umfasst,
wobei der Magnetfeldgenerator (120) einen Träger (40) umfasst und wobei die Spule (50) an den Träger (40) gebunden ist.

2. Einrichtung nach Anspruch 1, wobei eine Dicke der Spule (50) nicht mehr als 1 µm beträgt.

3. Einrichtung nach Anspruch 1 oder 2, wobei der Magnetfeldgenerator (120) weiter eine Steuereinheit (124) und eine Benutzerschnittstelle (125) für Benutzerbetrieb der Steuereinheit (124) umfasst.

4. Einrichtung nach Anspruch 3, wobei die Benutzerschnittstelle (125) einen Druckknopf umfasst, der sich an einer Außenseite der Einrichtung (100) befindet.

5. Einrichtung nach einem vorstehenden Anspruch, wobei der Magnetfeldgenerator (120) weiter einen Temperatursensor (126) umfasst.

6. Einrichtung nach einem vorstehenden Anspruch, wobei der Magnetfeldgenerator (120) weiter eine Schnittstelle (101) zum Zusammenwirken mit einem Artikel (1,2) umfasst.

7. Einrichtung nach Anspruch 6, wobei die Schnittstelle (101) eine Aussparung (101) umfasst, die konfiguriert ist, um den Artikel (1,2) über eine Öffnung (102) aufzunehmen.

8. Einrichtung nach einem vorstehenden Anspruch, wobei der Magnetfeldgenerator (120) eine elektrische Leistungsquelle (121) umfasst.

9. Einrichtung nach Anspruch 8, wobei die elektrische Leistungsquelle (121) eine aufladbare Batterie umfasst.

10. Einrichtung nach einem vorstehenden Anspruch, wobei der Träger (40) ein elektrischer Isolator ist.

11. Einrichtung nach einem vorstehenden Anspruch, wobei eine Baugruppe des Trägers (40) und des Films, der die Spule (50) definiert, flexibel sind, um imstande zu sein, zusammengedrückt zu werden, um eine Zylinderform zu bilden.

12. Einrichtung nach einem vorstehenden Anspruch, wobei die Spule (50) durch chemisches oder mechanisches Anhaften der Spule (50) an dem Träger (40) an den Träger (40) gebunden ist.

13. System, umfassend:
Einrichtung nach einem vorstehenden Anspruch; und
einen Artikel (1,2) zur Verwendung mit der Einrichtung, wobei der Artikel (1,2) das rauchbare Material (30) umfasst.

14. System nach Anspruch 13, wobei das rauchbare Material (30) ein tabakhaltiges Material umfasst.

15. System nach Anspruch 13 oder 14, wobei der Artikel (1,2) sich erhitzendes Material (10) umfasst, das durch Durchdringung mit dem sich verändernden Magnetfeld erhitzbar ist, um das rauchbare Material (30) zu erhitzen.

16. System nach Anspruch 15, wobei das sich erhitzende Material (10) ein Metall oder eine Metalllegierung umfasst.

17. System nach Anspruch 16, wobei das sich erhitzende Material (10) ein oder mehrere Materialien umfasst, die aus der Gruppe ausgewählt sind, bestehend aus: Aluminium, Gold, Eisen, Nickel, Kobalt, leitfähigem Kohlenstoff, Grafit, unlegiertem Kohlenstoffstahl, Edelstahl, ferritischem Edelstahl, Kupfer und Bronze.

## Revendications

1. Appareil permettant de chauffer un matériau à fumer pour volatiliser au moins un composant du matériau à fumer, l'appareil comprenant :
un générateur de champ magnétique (120) pour la génération d'un champ magnétique variable pour l'utilisation lors du matériau à fumer,
dans lequel le générateur de champ magnétique (120) comprend un film définissant une bobine (50) de matériau électroconducteur, et un dispositif (123) pour faire passer un courant électrique variable à travers la bobine (50),
dans lequel le générateur de champ magnétique (120) comprend un support (40) et dans lequel la bobine (50) est liée au support (40).

2. Appareil selon la revendication 1, dans lequel une épaisseur de la bobine (50) n'est pas supérieure à 1 µm.

3. Appareil selon la revendication 1 ou 2, dans lequel le générateur de champ magnétique (120) comprend en outre un dispositif de commande (124) et une interface utilisateur (125) pour l'actionnement par l'utilisateur du dispositif de commande (124).

4. Appareil selon la revendication 3, dans lequel l'interface utilisateur (125) comprend un bouton poussoir situé sur un extérieur de l'appareil (100).

5. Appareil selon une quelconque revendication précédente, dans lequel le générateur de champ magnétique (120) comprend en outre un capteur de température (126).

6. Appareil selon une quelconque revendication précédente, dans lequel le générateur de champ magnétique (120) comprend en outre une interface (101) pour coopérer avec un article (1, 2).

7. Appareil selon la revendication 6, dans lequel l'interface (101) comprend un évidement (101) configuré pour recevoir l'article (1, 2) via une ouverture (102).

8. Appareil selon une quelconque revendication précédente, dans lequel le générateur de champ magnétique (120) comprend une source d'énergie électrique (121).

9. Appareil selon la revendication 8, dans lequel la source d'énergie électrique (121) comprend une batterie rechargeable.

10. Appareil selon une quelconque revendication précédente, dans lequel le support (40) est un isolant électrique.

11. Appareil selon une quelconque revendication précédente, dans lequel un ensemble du support (40) et le film définissant la bobine (50) sont flexibles de sorte à être apte à être pressés pour présenter une forme cylindrique.

12. Appareil selon une quelconque revendication précédente, dans lequel la bobine (50) est liée au support (40) par adhésion chimique ou mécanique de la bobine (50) au support (40).

13. Système comprenant :
un appareil selon une quelconque revendication précédente ; et
un article (1, 2) destiné à être utilisé avec l'appareil, dans lequel l'article (1, 2) comprend le matériau à fumer (30).

14. Système selon la revendication 13, dans lequel le matériau à fumer (30) comprend un matériau contenant du tabac.

15. Système selon la revendication 13 ou 14, dans lequel l'article (1, 2) comprend du matériau chauffant (10) qui peut être chauffé par pénétration avec le champ magnétique variable pour chauffer le matériau à fumer (30).

16. Système selon la revendication 15, dans lequel le matériau chauffant (10) comprend un métal ou un alliage de métal.

17. Système selon la revendication 16, dans lequel le matériau chauffant (10) comprend un ou plusieurs matériaux sélectionnés dans le groupe consistant en : de l'aluminium, de l'or, du fer, du nickel, du cobalt, du carbone conducteur, du graphite, de l'acier au carbone ordinaire, de l'acier inoxydable, de l'acier inoxydable ferritique, du cuivre et du bronze.
